(19) Europäisches Patentamt
European Patent Office
Office européen des brevets

(11) Publication number: **0 400 805 A1**

(12) **EUROPEAN PATENT APPLICATION**

(21) Application number: **90304470.9**

(22) Date of filing: **25.04.90**

(51) Int. Cl.5: **C07D 501/20, C07D 501/22, C07D 501/36, A61K 31/545**

(30) Priority: **25.04.89 JP 106445/89**
**12.04.90 JP 97483/90**

(43) Date of publication of application:
**05.12.90 Bulletin 90/49**

(84) Designated Contracting States:
**AT BE CH DE DK ES FR GB IT LI NL SE**

(71) Applicant: **Ishimaru, Toshiyasu**
**1-12-11 Daiwa-higashi**
**Kawanisha-shi, Hyoga-ken(JP)**

(72) Inventor: **The designation of the inventor has not yet been filed**

(74) Representative: **Lawrence, Malcolm Graham et al**
**Hepworth, Lawrence & Bryer 2nd Floor, Gate House South Westgate Road**
**Harlow Essex CM20 1JN(GB)**

(54) **Cephalosporin compounds and their use.**

(57) A cephalosporin compound of the formula (I):

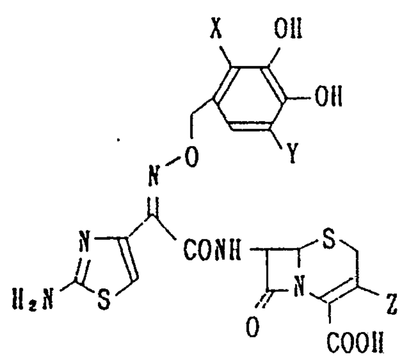

( I )

wherein X and Y may be the same or different and are a hydrogen or halogen atom; Z is a hydrogen or halogen atom, $-CH=CH_2$, $-CH=CH-CH_3$, $-OR^1$, $-SR^2$ or $-CH_2W$ in which $R^1$ and $R^2$ are a lower alkyl group and W is a hydrogen atom or a nucleophilic residue, its pharmaceutically acceptable salt, a process for preparing the same and their antibacterial composition.

EP 0 400 805 A1

## Cephalosporin Compounds and Their Use

This invention relates to novel cephalosporin compounds possessing antibacterial activity, their composition and a process for preparing the same.

Cephalosporin antibiotics generally show a broad spectrum of antibacterial activities against Gram-positive and Gram-negative bacteria and hence various cephalosporin compounds have been clinically used as therapeutic agents for a variety of infectious diseases. However, there are not many therapeutic agents of cephalosporin compounds which show potent activity against Pseudomonas strains and Proteus strains. Also, many cephalosporin compounds have the drawbacks that they are unstable for β-lactamase which is produced by a resistant strain and are low in anti-bacterial activity against Pseudomonas strains which are presently clinical problem ("Cephalosporins and Penicillins, Chemistry and Biology" edited by E. H. Flynn, Academic Press, New York, N.Y., 1972, Chapter II).

Among various acyl groups at 7th position of cephem compounds, the following has been disclosed in Japanese Unexamined Publication Nos. 62(1987)-270589 and 63(1988)-99077.

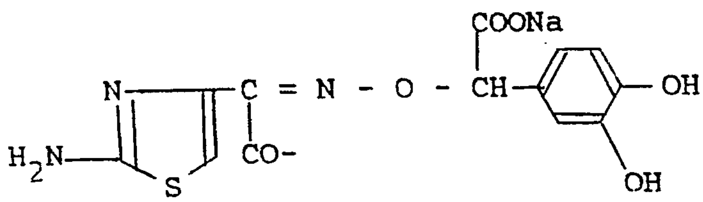

The inventors have extensively studied on the relationship between chemical structures of 7th and 3rd substituents in cephem compounds and their activities. As the result, we found that new cephalosporin compounds of the following formula (I) show a broad spectrum of antibacterial activity against Gram-positive and Gram-negative strains, especially a potent activity against Gram-positive strains, Pseudomonas strains and β-lactamase producing strains and are well absorbed with low toxicity.

According to this invention, it provides a cephalosporin compound of the formula (I):

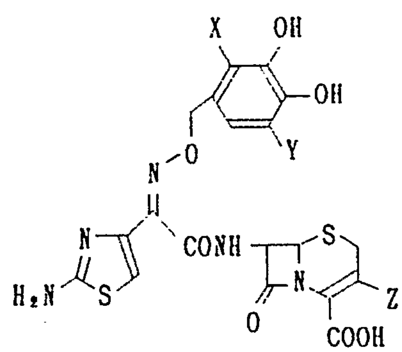

( I )

wherein X and Y may be the same or different and are a hydrogen or halogen atom; Z is a hydrogen or halogen atom, $-CH=CH_2$, $-CH=CH-CH_3$, $-OR^1$, $-SR^2$ or $-CH_2W$ in which $R_1$ and $R_2$ are a lower alkyl group and W is a hydrogen atom or a nucleophilic residue, or its pharmaceutically acceptable salt.

Also, it provides an antibacterial composition comprising an effective amount of the compound (I) or its pharmaceutically acceptable salt and a pharmaceutically acceptable carrier or diluent.

Examples of the pharmaceutically acceptable salts of the compounds (I) include conventional non-toxic salts, such as alkali metal salts (e.g., sodium or potassium salt), alkali earth metal salts (e.g., calcium or magnesium salts), ammonium salt and salts with organic bases (e.g., triethylamine salt, pyridine salt, ethanolamine salt, triethanolamine salt, dicyclohexylamine salt, lysine salt or arginine salt).

The halogen atom in the definitions of X and Y of the compounds (I) includes chlorine, bromine and fluorine atoms. Preferably, the halogen atom is chlorine or fluorine atom. More preferably, the halogen atom is chlorine atom.

It is preferable that both of X and Y are hydrogen atom, chlorine atom or fluorine atom, or any one of X and Y is chlorine atom or fluorine atom and the remaining one is hydrogen atom.

The lower alkyl group of the group $-OR^1$ or $-SR^2$ means a straight-chain or branched-chain alkyl group containing 1 - 4 carbon atoms, specifically methyl, ethyl, propyl, isopropyl, butyl and isobutyl.

The nucleophilic residue of W in the group $-CH_2W$ includes a halogen atom, an optically substituted aromatic heterocycle-thio group, a lower alkylthio group, a lower alkoxy group, a primary, secondary or tertiary amino group or a heterocyclic group containing at least one nitrogen group to be bonded to the methylene group via the nitrogen atom. The halogen atoms mentioned in X and Y are applicable to the above halogen atom. The aromatic heterocycle in the optionally substituted aromatic heterocyclo-thio group includes a five or six membered heterocycle containing at least one hetero atom of nitrogen, sulfur and oxygen atoms, specifically thiazole, isothiazole, thiadiazole, triazole, tetrazole, pyridine, pyrimidine, pyridazine, oxazole, imidazole, pyrazole, oxadiazole, isoxazole and triazine. Examples of the substituents on the aromatic heterocycle are a lower alkyl group such as methyl, ethyl or propyl, a halogen atom such as chlorine, bromine or fluorine, amino group and cyano group.

Examples of the lower alkylthio group are methylthio, ethylthio and propylthio. Examples of the lower alkoxyl groups are methoxyl, ethoxyl and propoxyl. Examples of the primary, secondary or tertiary amino groups are amino groups substituted by one to three of a lower alkyl or benzyl; specifically methylamino, ethylamino, dimethylamino, triethylamino and methylbenzylamino.

The heterocyclic groups containing at least one nitrogen atom to be bonded to the methylene group via the nitrogen atom include such heterocyclic groups containing nitrogen atom(s) as mentioned in the above aromatic heterocycle. The heterocylic groups may be substituted by a lower alkyl such as methyl or ethyl; a halogen such as chlorine, bromine or fluorine, amino or cyano. Preferably, the neucleophilic residue is (1,2,3-thiadiazol-5-yl)thio, (5-methyl-2-tetrazol-2-yl)thio or (N-methylpyridin-4-yl)thio.

The compound (I) of this invention can be prepared by reacting a compound of the formula (II):

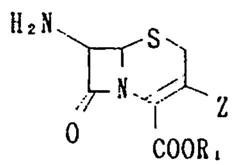

( II )

wherein Z is the same meaning as defined in the formula (I), $R_1$ is a hydrogen atom or a protecting group for the carboxyl group,
or its reactive derivative at the amino group or salt,
with a compound of the formula (III):

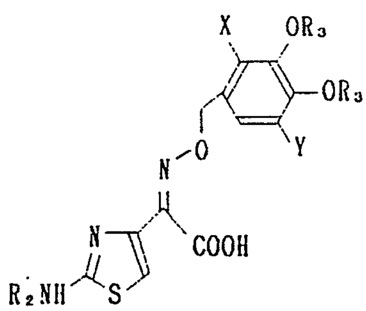

( III )

, wherein X and Y have the same meanings as defined in the formula (I), $R_2$ is a hydrogen atom or a protecting group for amino group and $R_3$ is a hydrogen atom or a protecting group for hydroxyl group, or its reactive derivative at the carboxyl group or salt.

Examples of suitable reactive derivatives at the amino group of the compounds (II) are an imino derivative of Schiff's base type formed by the reaction of the compound (II) and a carbonyl compound such as an aldehyde or ketone, or its enamine type isomer, a silyl derivative formed from the compound (II) and a silylating agent such as bis(tri-methylsilyl)acetamide, or a derivative formed from the compound (II) and phosphorus trichloride or phosgene.

Suitable salts of the compounds (II) and (III) are acid addition salts with organic acids such as acetic acid, maleic acid, tartaric acid, benzenesulfonic acid and toluenesulfonic acid; or acid addition salts with inorganic acids such as hydrochloric acid, hydrobromic acid, sulfuric acid and phosphoric acid; metal salts

such as alkali metal or alkaline earth metal salts (e.g., sodium, potassium, calcium or magnesium salt); ammonium salt and salts with organic amines such as triethylamine and dicyclohexylamine.

Examples of suitable reactive derivatives at the carboxyl group of the compounds (III) include the acid halides, acid azide, mixed anhydrides, active amides and active esters. Specifically, the acid halide may be the acid chloride and acid bromide; the mixed acid anhydride may be those formed with an acid such as a substituted phosphoric acid (e.g., a dialkyl phosphate, dibenzyl phosphate or a halogenated phosphoric acid), dialkyl phosphite, sulfurous acid, thiosulfuric acid, sulfuric acid, an alkyl carbonate (e.g., methyl carbonate or ethyl carbonate), an aliphtic carboxylic acid (e.g., pivalic acid, valeric acid, isovaleric acid, 2-ethylacetic acid or trichloroacetic acid) or an aromatic carboxylic acid (e.g., benzoic acid); the active amide may be the active amide formed with imidazole, dimethylpyrazole, triazole or tetrazole; the active ester may be the cyanomethyl ester, methoxymethyl ester, dimethyliminomethyl[$(CH_3)_2N=CH$-] ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenylthio ester, p-nitrophenylthio ester, p-cresylthio ester, carboxymethylthio ester, pyranyl ester, pyridyl ester, piperidyl ester, 8-quinolylthio ester or an ester formed with a N-hydroxy compound (e.g., N,N-dimethylhydroxyamine, 1-hydroxy-2-(1H)-pyridone, N-hydroxysuccinimide, N-hydroxyphthalimide or 1-hydroxy-6-chloro-1H-benzotriazole). The reactive derivative is suitably selected and used, depending upon the kind of the compound (III) as the reactant.

The reaction of the compound (II) and the compound (III) is usually conducted in a conventional solvent such as water, acetone, dioxane, acetonitrile, chloroform, methylene chloride, tetrahydrofuran, ethyl acetate, N,N-dimethylformamide or pyridine, or other organic solvent which does not impede the reaction. The organic solvents as mentioned above may be used as a mixture with water.

When the compound (III) is used in the free acid form or salt form, the reaction is preferably conducted in the presence of a condensing agent, such as N,N′-dicyclohexylcarbodiimide, N-cyclohexyl-N′-morpholinoethylcarbodiimide, N-cyclohexyl-N′-(4-diethylaminocyclohexyl)carbodiimide, N,N′-diethylcarbodiimide, N,N′-diisopropylcarbodiimide, N-ethyl-N′-(3-dimethylaminopropyl)carbodiimide, N,N′-carbonylbis-(2-methylimidazole), pentamethyleneketene-N-cyclohexylimine, diphenylketene, N-cyclohexylimine, ethoxyacetylene, 1-alkoxy-1-chloroethylene, trialkyl phosphite, ethyl polyphosphate, isopropyl polyphosphate, phosphorus oxy chloride, phosphorus trichloride, thionyl chloride, oxalyl chloride, triphenylphosphine, 2-ethyl-7-hydroxybenzisoxazolium salt, 2-ethyl-5-(m-sulfophenyl)isoxazolium hydroxide inner salt, 1-(p-chlorobenzenesulfonyloxy)-6-chloro-1H-benzotriazole, or Vilsmeyer's reagent which is produced by the reaction of dimethylformamide with thionyl chloride, phosgene or phosphorus oxychloride.

The above reaction can be also conducted in the presence of an inorganic or organic base, especially such as an alkali metal hydrogen carbonate (e.g., sodium hydrogen carbonate or potassium hydrogen carbonate), an alkaline earth metal carbonate (e.g., potassium carbonate), a tri-lower alkylamine (e.g., triethylamine or trimethylamine), pyridine, a N-lower alkylmorpholine or a N,N-dilower alkylbenzylamine.

The reaction is usually conducted under cooling or warming, without limitation thereto.

Alternatively, the compound (I) can be prepared by reacting a compound of the formula (IV):

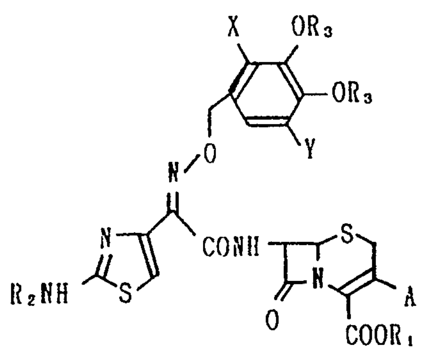

(IV)

wherein A is acetoxyl group or a halogen atom, and X, Y, $R_1$, $R_2$ and $R_3$ have the same meanings as defined above,

with a nucleophilic compound.

The nucleophilic compounds to be used include e.g., an optionally substituted heterocyclic thiol containing at least one of sulfur, nitrogen and oxygen atoms, a lower alkylthiol, a lower alkanol, a primary, secondary or tertiary amine or a heterocyclic compound containing at least one nitrogen atom.

When the above reaction gives a protected derivative at the amino, carboxyl and/or phenolic hydroxyl groups of the compound (I), such protected derivative if necessary is subjected to a removal for protecting

group in accordance with a conventional method. Appropriate method for the removal reaction is selected and used depending upon the kinds of the protecting groups. For example, the removal for amino-protecting group is usually conducted by hydrolysis or reduction but acyl group as the amino-protecting group can be removed e.g., by the treatment with an imino-halogenating agent and then with an imino-etherizating agent, followed by hydrolysis. A hydrolysis using an acid is conventional for amino-protecting group and is applicable to remove alkoxycarbonyl, formyl, trityl or like group. Examples of the acids are formic acid, trifluoroacetic acid, hydrochloric acid or like acid, which is suitably selected upon the kind of amino-protecting group. The removal for amino-protecting group can be generally conducted without solvent or in the presence of water, a hydrophilic organic solvent or a mixture thereof. When trifluoroacetic acid is used, the removal may be conducted in the presence of anisole. Also, the removal for carboxyl-protecting group is usually conducted by a conventional method such as hydrolysis or reduction. A hydrolysis using an acid is conventional and applicable to silyl group, p-methoxybenzyl group, diphenylmethyl group or like group. The removal for phenolic hydroxy-protecting group is also conducted by any conventional method such as hydrolysis or reduction. For the hydrolysis, an acid or base is used. For example, p-methoxybenzyl or diphenylmethyl group can be removed by a hydrolysis using an acid, and acetyl, benzoyl or like acyl group be removed by a hydrolysis using a base.

When the compound (I) is obtained in the free form at 3rd carboxyl group, it can be converted into the corresponding salt in accordance with the conventional method. On the other hand, a salt of the compound (I) when produced can be also converted into the corresponding free form.

The compounds (II) as produced can be purified and isolated by the conventional methods, e.g., purification using adsorptive resins [e.g., Amberlite® XAD-2 (Rohm & Haas), Diaion® HP-20 or Sepabeads® SP207 (Mitsubishi Chem., Japan)], precipitation, crystallization and a combination thereof.

The compounds (I) or their salts of the invention are valuable antibiotics showing potent antibacterial activities against broad pathogenic bacteria including Gram-positive and Gram-negative strains and can be safely used as an antibacterial agent for treating or preventing infections caused by the above bacteria in man or domestic animals.

The compounds (I) or their salts may be used in various preparations such as parenteral preparations (e.g., intravenous or intramascular injections), oral preparations (e.g., capsules, tablets or powders) or rectal preparations (e.g., oily or water-soluble suppositories). Such various preparations can be prepared by the conventional methods, using diluent, excipients, binders, wetting agents, disintegrating agents, surfactants, lubricants, dispersing agents, buffering agents, preservatives, solubilizers, antiseptics, correctives and/or soothing agents.

Daily dose of the compound (I) or its salt may depend upon the conditions and weights of the patients, administration routes and the like. For parenteral administration, it is suitably about 250 - 3000 mg for adult in one to four divided doses per day.

The invention will be further illustrated by Reference Examples and Examples.

In these Examples, unless specific attention is given, NMR was measured by use of 90 MHz, $\delta$ value in $D_2O$ bases on 4.82 as $\delta$ value for water peak and $\delta$ value in other deutrium solvents bases on TMS as the internal standard. In the formula, PMB means p-methoxybenzyl and $Ph_3C$ is trityl.

Reference Example 1

i) <u>2,5-Dichloro-3,4-dihydroxybenzoic acid</u>

In 200ml of acetic acid were added 50g of 3,4-dihydroxybenzoic acid (protocatechuic acid) and 105.1g of sulfuryl chloride. The mixture was stirred for 10 hours at 50 °C, and then was cooled to crystallize the product. The crude crystals were recrystallized from ethyl acetate and n-hexane to obtain 35g of the title compound (mp. 230 °C).

ii) <u>p-Methoxybenzyl 2,5-dichloro-3,4-di-p-methoxybenzyloxybenzoate</u>

To a solution of 10g of 2,5-dichloro-3,4-dihydroxybenzoic acid in 50ml of dimethylformamide (DMF) were added 28g of p-methoxybenzyl chloride and 33g of potassium carbonate. The mixture was stirred for 10 hours at 70 - 80 °C. After the completion of the reaction, 200ml of ethyl acetate were added to the reaction mixture and insoluble material was removed by filtration. The filtrate was distilled under reduced

pressure to remove the solvent and DMF. To the residue were added ethyl acetate and saline. The organic phase as collected was washed with water, dried over anhydrous $MgSO_4$ and distilled under reduced pressure to remove the solvent. The residue was purified by silica gel column chromatography (Wako-gel® C-200 : 700g, developing solvent : benzene and ethyl acetate (20:1)], to obtain 18g of the title compound.

iii) 2,5-Dichloro-3,4-di-p-methoxybenzyloxybenzylalcohol

$LiAlH_4$ (2.7g) was suspended in 100ml of anhydrous tetrahydrofuran and ice-cooled. A solution of 10g of p-methoxybenzyl 2,5-dichloro-3,4-di-p-methoxybenzyloxybenzoate in 10ml of anhydrous tetrahydrofuran was dropwise added to the above cooled suspension and stirred for 6 hours under ice-cooling. After the completion of the reaction, the reaction mixture to which 10ml of ethyl acetate were added was stirred for 30 minutes and adjusted to pH 2.0 by addition of 20% citric acid under ice-cooling. The mixture was filtered to remove insoluble material, and the filtrate was poured into 300ml of ethyl acetate. The mixture was washed with water, dried over anhydrous $MgSO_4$ and distilled under reduced pressure to remove the solvent. The residue was purified by silica gel column chromatography [Wako-gel® C-200: 100g, developing solvent : benzene and ethyl acetate (20:1)] to obtain 7g of the title compound.
NMR($CDCl_3$, δ, ppm)
3.85(6H,s), 4.55(2H,bs), 4.70(2H,bs), 5.00(2H,s), 6.85-6.90(4H,d), 7.20-7.35(5H,m)

iv) Allyl 2-(2-tritylaminothiazol-4-yl)-2(Z)-[2,5-dichloro-(3,4-di-p-methoxybenzyloxybenzyloxy)imino]acetate

2,5-Dichloro-(3,4-di-p-methoxybenzyloxy)bezylalcohol (2g) was dissolved in 20ml of methylene chloride and cooled to -10°C. To the solution was added 573mg of 2,6-lutidine and then dropwise added a solution of 632mg of thionyl chloride in 5ml of methylene chloride. The mixture was stirred for an hour at the above temperature, washed with water, dried over anhydrous $MgSO_4$ and distilled under reduced pressure to remove the solvent.

The resulting residue and 1g of $K_2CO_3$ were added to a solution of 2g of allyl 2-(2-tritylaminothiazol-4-yl)-2(Z)-hydroxyiminoacetate in 10ml of DMF, followed by stirring for 20 hours at room temperature. The reaction mixture was poured into 100ml of ethyl acetate, and the mixture was washed with water, dried over anhydrous $MgSO_4$ and distilled to remove the solvent. The residue was purified by silica gel column chromatography [Wako-gel® C-200: 30g, developing solvent: benzene and ethyl acetate (20:1)] to afford 3.2g of the title compound.
IR (nujol) 3300 - 3200, 1720cm$^{-1}$
NMR($CDCl_3$, δ value ppm)
3.8(3H,s), 3.81(3H,s), 5.0(2H,s), 5.05(2H,s), 5.1(2H,s), 5.8(2H,d), 6.1(1H,m), 6.7-6.9 (7H,m), 7.3(19H,m)

v) 2-(2-Tritylaminothiazol-4-yl)-2(Z)-(2,5-dichloro-3,4-di-p-methoxybenzyloxybenzyloxyimino)acetic acid

To an ice-cooled solution of 900mg of allyl 2-(2-tritylaminothiazol-4-yl)-2(Z)-(2,5-dichloro-3,4-di-p-methoxybenzyloxybenzyloxyimino)acetate in 20ml of methylene chloride were added 183mg of sodium 2-ethylhexanoate and 50mg of tetrakistriphenylphosphine-palladium complex, followed by stirring for an hour under ice-cooling. The reaction mixture was adjusted to pH 2.0, washed with water, dried over anhydrous $MgSO_4$ and distilled under reduced pressure to obtain 620mg of the title compound.
NMR($CDCl_3$, δ value ppm)
3.75(3H,s), 3.80(3H,s), 5.0(2H,s), 5.05(2H,s), 5.06, 5.10(2H,ABq,J=12Hz), 6.75-6.90(7H,m), 7.25-7.35(19H,m)

Reference Example 2

i) Allyl 2-(2-tritylaminothiazol-4-yl)-2(Z)-(3,4-di-p-methoxybenzyloxybenzyloxyimino)acetate

To an ice-cooled solution of 470mg of allyl 2-(2-tritylaminothiazol-4-yl)-2(Z)-hydroxyiminoacetate in 5ml of DMF were added 207mg of calcium carbonate and 500mg of 3,4-di-p-methoxybenzyloxybenzyl chloride. The mixture was stirred for 20 hours at room temperature, and then poured into 60ml of ethyl acetate. The

mixture was washed with water, dried over anhydrous $MgSO_4$ and distilled under reduced pressure to remove the solvent. The residue was purified by silica gel column chromatography [Wako-gel® C-200: 20g, developing solvent : benzene and ethyl acetate (20:1)] to obtain 450mg of the title compound.
IR (nujol) 1720cm$^{-1}$
NMR($CDCl_3$, $\delta$ value, ppm)
3.75(3H,s), 3.80(3H,s), 4.95(2H,s), 5.00(2H,s), 5.05(2H,bs), 5.80(2H,b,d), 6.00(1H,m), 6.70-6.95(9H,m), 7.30-7.50(9H,m)

ii) 2-(2-Tritylaminothiazol-4-yl)-2(Z)-(3,4-di-p-methoxybenzyloxybenzyloxyimino)acetic acid

To an ice-cooled solution of 780mg of allyl 2-(2-tritylaminothizol-4-yl)-2(Z)-(3,4-di-p-methoxybenzyloxybenzyloxyimino)acetate in 20ml of methylene chloride were added 190mg of sodium 2-ethylhexanoate and 50mg of tetrakistriphenylphosphine-palladium complex. The mixture was stirred for an hour under ice-cooling, poured into water and adjusted to pH 2.0. The organic phase as collected was washed with water, dried over anhydrous $MgSO_4$ and distilled under reduced pressure to remove the solvent. The residue was washed with isopropylether and dried to obtain 610mg of the title compound.
IR (nujol) 1600cm$^{-1}$
NMR($CDCl_3$, $\delta$ value, ppm)
3.75(9H,s), 3.80(3H,s), 4.95(2H,s), 5.00(2H,s), 5.06, 5.10(2H,ABg, J=2Hz), 6.75-6.90(9H,m), 7.25-7.35(9H,m)

Reference Example 3

i) Methyl 5-chloro-3,4-dihydroxybenzoate

A solution of 5g of 5-chloro-3,4-dihydroxybenzoic acid in 40ml of anhydrous methanol was cooled to -20° C to -10° C, saturted with dry hydrogen chloride gas, and then allowed to stand for 20 hours at room temperature. After adding a little amount of benzene, the reaction mixture was concentrated in about 1/3 volume and ice-cooled. The precipitated crystals were washed with a mixture of n-hexane and methanol to obtain the title compound. The mother liquor was also treated to obtain second crystals. Yield 4.9g (92%). mp 202 - 203° C (methanol).

ii) Methyl 5-chloro-3,4-di-p-methoxybenzyloxybenzoate

To a solution of 4.46g of methyl 5-chloro-3,4-dihydroxybenzoate in 16ml of DMF were added 7g of potassium carbonate and 9.5g of p-methoxybenzyl chloride at room temperature. The mixture was stirred for 20 hours. After the completion of the reaction, 30ml of ethyl acetate were added to the reaction mixture and insoluble material was removed by filtration. To the filtrate were added 30ml of ethyl acetate and 20ml of water, and the mixture was adjusted to pH 7.3 by addition of 7% aqueous sodium hydrogen carbonate under ice-cooling. The organic layer was collected, while the aqueous layer was washed several times with ethyl acetate. The combined orgnaic layers were washed with water, dried over anhydrous $MgSO_4$ and concentrated under reduced pressure. The residue was crystallized with ethyl acetate-n-hexane to obtain 8.95g (91%) of the title compound as colorless crystals. mp 122 - 123° C (methanol).

iii) 5-Chloro-(3,4-di-p-methoxybenzyloxy)benzylalcohol

Methyl 5-chloro-3,4-di-p-methoxybenzyloxybenzoate (6.42g) as obtained by the above ii) was treated by the same method of Reference Example 1, iii) to obtain 5.58g of the title compound (94%). mp 107° C

iv) Allyl 2-(2-tritylaminothiazol-4-yl)-2(Z)-(5-chloro-3,4-di-p-methoxybenzyloxybenzyl)oxyiminoacetate

A solution of 0.65g of trichloromethyl chloroformate in 1 ml of methylene chloride was dropwise added to a mixture of 0.55g of DMF and 40ml of methylene chloride cooled to -15° C. The mixture was gradually

warmed to 20 to 30° C, stirred for 30 minutes at the same temperature and again cooled to -20° C. To the solution were added 2.65g of 5-chloro-3,4-di-p-methoxybenzyloxybenzylalcohol, followed by stirring for 40 minutes at -20 to -10° C. The reaction mixture was poured into 20ml of ice-water and stirred. The aqueous layer was extracted with methylene chloride several times. The combined orgnaic layers were washed with water, dried over anhydrous $MgSO_4$ and concentrated under reduced pressure.

The residue was dissolved in a mixture of 4ml of DMF and 6ml of DMSO, to which 3.3g of allyl 2-(2-tritylaminothiazol-4- yl)-2(Z)-hydroxyiminoacetate (mp 187 - 189° C) and 1.4g of potassium carbonate were added and stirred for 20 hours at room temperature. After the completion of the reaction, 30ml of ethyl acetate were added to the reaction mixture. Insoluble material was filtered off, and 30ml of ethyl acetate and 20ml of water were added to the filtrate. The mixture was adjusted to pH 7.3 by adding 7% aqueous sodium hydrogen crabonate under ice-cooling and stirring. The organic layer was collected while the aqueous layer was extracted with ethyl acetate several times. The combined organic layers were washed with water, dried over anhydrous $MgSO_4$ and concentrated under reduced pressure. The residue was purified by silica gel column chromatography (eluting with ethyl acetate and benezene) to obtain 4.16g (74%) of the title compound as powders.

v) <u>(Z)-2-(2-tritylaminothiazol-4-yl)-2(Z)-(5-chloro-3,4-di-p-methoxybenzyloxybenzyl)oxyiminoacetic acid</u>

To a solution of 3g of the allyl ester as obtained in the above iv) in 20ml of methylene chloride was added a solution of 1.5g of potassium 2-ethylhexanoate in 8ml of ethyl acetate under nitrogen gas atmosphere, and then added 90mg of triphenylphsphine and 50mg of tetrakistriphenylphoshine-palladium complex, followed by stirring for 3 hours at room temperature. Then, 50ml of isopropyl ether were added to the mixture, stirred for an hour and ice-cooled. The precipitated crystals were washed with a mixture of ethyl acetate and isopropyl ether. Thus, potassium salt of the title compound was obtained. The potassium salt was mixed with 30ml of methylene chloride and 10ml of water and the mixture was adjusted to pH 2.5 by adding citric acid under ice-cooling and stirring. The organic layer was sepearted while the aqueous layer was extracted with ethyl acetate several times. The combined organic layers were washed with water, dried over anhydrous $MgSO_4$ and concentrated under reduced pressure. The residue was recrystallized with n-hexane to obtain 2.2g (77%) of the title compound as colorless crystals (mp 122 - 126° C).

Example 1

A: p-Methoxybenzyl 7-[2-(2-tritylaminothiazol-4-yl)-2(Z)-(2,5-dichloro-3,4-di-p-methoxybenzyloxybenzyloxyimino)acetamido]-3-(1,2,3-thiadiazol-5-yl)thiomethyl-3-cephem-4-carboxylate

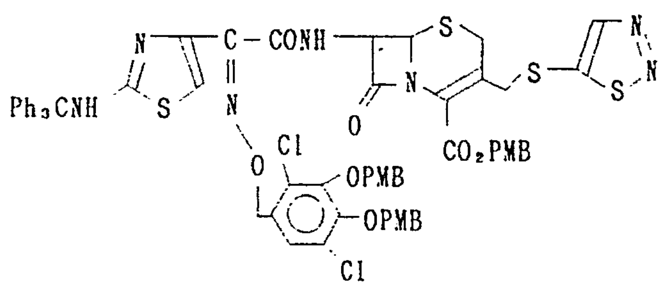

(PMB is p-methoxybenzyl group and $Ph_3C$ is trityl group.)

2-(2-Tritylaminothiazol-4-yl)-2(Z)-(2,5-dichloro-3,4-di-p-methoxybenzyloxybenzyloxyimino)acetic acid (860mg) and p-methoxybenzyl 7-amino-3-(1,2,3-thiadiazol-5-yl)thiomethyl-3-cephem-4-carboxylate (450mg) were dissolved in 20ml of methylene chloride and cooled to -20° C. To the solution were added 320ml of pyridine and then dropwise added a solution of 220mg of phosphorus oxychloride in 5ml of methylene chloride, taking about 5 - 10 minutes. The mixture was stirred for an hour at 0 to 5° C and poured into ice-water. Then, the mixture was adjusted to pH 7.0 by addition of 7% aqueous $NaHCO_3$ solution and stirred for 30 minutes at 5° C. The organic phase was washed with water, dried over anhydrous $MgSO_4$ and distilled under reduced pressure to remove the solvent. The residue was purified by silica gel column chromatography [Wako-gel® C-200: 30g; developing solvent : benzene and ethyl acetate (10:1)] to obtain 750mg of the title compound.
IR (nujol) 3200, 1780, 1720, 1680, 1620cm$^{-1}$
NMR($CDCl_3$, δ value, ppm)
3.30,3.51(2H,ABq,J=18Hz), 3.80(3H,s,$OCH_3$), 3.82(6H,s), 3.93,4.13(2H,ABq,J=13Hz), 4.95-4.98(6H,m), 5.18-(1H,d,J=4.8Hz), 5.36(2H,bl), 5.86(1H,d,d, J=5Hz and 10Hz), 6.75-7.10(9H,m), 7.25-7.40(21H,m), 8.40(1H,s)

B: Sodium 7-[2-(2-aminothiazol-4-yl)-2(Z)-(2,5-dichloro-3,4-dihydroxybenzyloxyimino)acetamido]-3-(1,2,3-thiadiazol-5-yl)thiomethyl-3-cephem-4-carboxylate

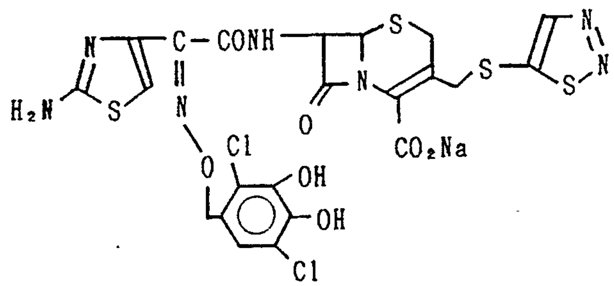

To an ice-cooled solution of 200mg of p-methoxybenzyl 7-[2-(2-tritylaminothiazol-4-yl)-2(Z)-(2,5-dichloro-3,4-di-p-methoxybenzyloxyimino)acetamido]-3-(1,2,3-thiadiazol-5-yl)thiomethyl-3-cephem-4-carboxylate in 0.5ml of anisole was added 2ml of trifluoroacetic acid. The mixture was stirred for an hour under ice-cooling. After the completion of the reaction, 50ml of isopropyl ether were added to the reaction mixture, and the precipitate as trifluoroacetate of the title compound was collected by filtration. After drying, the trifluoroacetate was suspended in 1ml of water, to which 7% $NaHCO_3$ was added to adjust to pH 7.2. The resulting solution was subjected to a column (Diaion® HP-20, 20ml), eluting with 5% aqueous acetone. The eluate was distilled to remove the solvent. The residue was freeze-dried to obtain 40mg of the title compound.
IR(nujol) 3400-3200, 1760, 1650, 1600cm$^{-1}$
NMR(DMSO-$d_6$, δ value, ppm)
3.60(2H,s), 4.40(2H,s), 4.98(1H,d,J=5Hz), 5.05(2H,b,s), 5.56(1H,d,d,J=5Hz and 10Hz), 6.74(1H,s), 6.88-(1H,s), 7.20(2H,b.s), 9.04(1H,s), 9.60(1H,d,J=10Hz)

Example 2

A: p-Methoxybenzyl 7-[2-(3-tritylaminothiazol-4-yl)-2(Z)-(2-chloro-3,4-di-p-methoxybenzyloxybenzyloxyimino)acetamido]-3-(1,2,3-thiadiazol-5-yl)thiomethyl-3-cephem-4-carboxylate

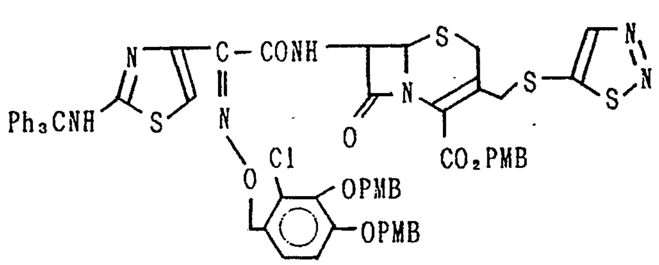

By the same method as in Example 1, the title compound (680mg) was obtained from 825mg of 2-(2-tritylaminothiazol-4-yl)-2(Z)-(2-chloro-3,4-di-p-methoxybenzyloxybenzyloxyimino)acetic acid and 450mg of 7-amino-3-(1,2,3-thiadiazol-5-yl)thiomethyl-3-cephem-4-carboxylic acid.
IR(nujol) 3200, 1780, 1710, 1680, 1610cm$^{-1}$

NMR($CDCl_3$, δ value, ppm)
3.23,3.46(2H,ABq,J = 18Hz), 3.79(3H,s), 3.80(6H,s), 4.06,4.12(2H,ABq,J = 14Hz), 4.91(1H,d,J = 5Hz), 5.12-(4H,m), 5.25(2H,bs), 5.81(1H,dd,J = 5Hz and 9Hz), 6.74-6.98(9H,m), 7.25-7.35(22H,m), 8.44(1H,s)

B. Sodium 7-[2-(2-aminothiazol-4-yl)-2(Z)-(2-chloro-3,4-dihydroxybenzyloxyimino)acetamido]-3-(1,2,3-thiadiazol-5-yl)thiomethyl-3-cephem-4-carboxylate

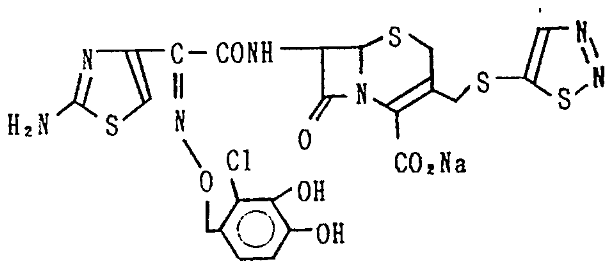

By the same method as in Example 1, the title compound (32mg) was obtained from 150mg of p-methoxybenzyl 7-[2-(2-tritylaminothiazol-4-yl)-2(Z)-(2-chloro-3,4-di-p-methoxybenzyloxybenzyloxyimino)-acetamideo]-3-(1,2,3-thiadiazol-5-yl)thiomethyl-3-cephem-4-carboxylate.
IR (nujol) 3400-3200, 1780, 1680, 1610cm$^{-1}$
NMR(DMSO-$d_6$, δ value, ppm)
3.73(2H,s), 4.41(2H,s), 4.98(1H,d,J = 5Hz), 5.03(2H,s), 5.54(1H,dd,J = 4.8Hz and 8.2Hz), 6.70(1H,s), 6.87-(1H,d,J = 8.6Hz), 7.17(2H,bs), 7.30(1H,d,J = 8.6Hz), 9.06(1H,s), 9.55(1H,d,J = 8.6Hz)

Example 3

A. p-Methoxybenzyl 7-[2-(2-tritylaminothiazol-4-yl)-2(Z)-(2,5-dichloro-3,4-di-p-methoxybenzyloxybenzyloxyimino)acetamido]-3-chloromethyl-3-cephem-4-carboxylate

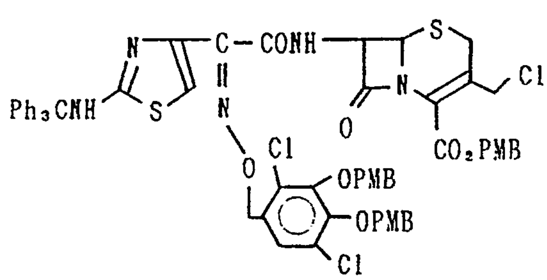

By the same method of Example 1, the title compound (850mg) was obtained from 860mg of 2-(2-tritylaminothiazol-4-yl)-2(Z)-(2,5-dichloro-3,4-di-p-methoxybenzyloxybenzyloxyimino)acetic acid and 370mg of p-methoxybenzyl 7-amino-3-chloromethyl-3-cephem-4-carboxylate.
IR (nujol) 3300, 1780, 1680, 1610cm$^{-1}$
NMR($CDCl_3$, δ value, ppm)
3.36,3.58(2H,ABq,J = 18Hz), 3.79(3H,s), 3.82(6H,s), 4.32,4.53(2H,ABq,J = 12Hz), 5.92-5.00(6H,m), 5.18-(1H,d,J = 4.8Hz), 5.37(2H,bs), 5.91(1H,dd,J = 4.8Hz and 9Hz), 6.75-6.90(9H,m), 7.30-7.40(21H,m)

B. p-Methoxybenzyl 7-[2-(2-tritylaminothiazol-4-yl)-2(Z)-(2,5-dichloro-3,4-di-p-methoxybenzyloxybenzyloxyimino)acetamido]-3-(N-methylpyridin-4-yl)thiomethyl-3-cephem-4-carboxylate•iodide

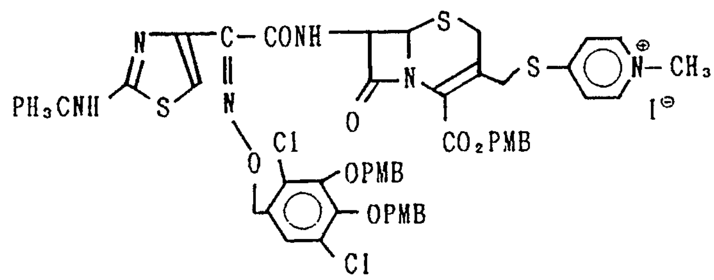

p-Methoxybenzyl 7-[2-(2-tritylaminothiazol-4-yl)-2(Z)-(2,5-dichloro-3,4-di-p-methoxybenzyloxybenzyloxyimino)acetamide]-3-chloromethyl-3-cephem-4-carboxylate (150mg) as obtained by the above A was dissolved in 5ml of tetrahydrofuran and ice-cooled. To the solution were added 20mg of sodium iodide and 20mg of N-methyl-4-pyridothione, followed by stirring for 4 hours under nitrogen gas atmosphere and under ice-cooling. After the completion of the reaction , the reaction mixture was poured in to 50ml of methylene chloride. The resulting mixture was washed with water, dried over anhydrous $MgSO_4$ and distilled under reduced pressure to remove the solvent. The residue was washed with isopropyl ether and then ethyl acetate to obtain 150mg of the title compound.
IR (nujol) $1760cm^{-1}$

C. 7-[2-(2-aminothiazol-4-yl)-2(Z)-(2,5-dichloro-3,4-di hydroxybenzyloxyimino)acetamido]-3-(N-methylpyridin-4-yl)thiomethyl-3-cephem-4-carboxylic acid

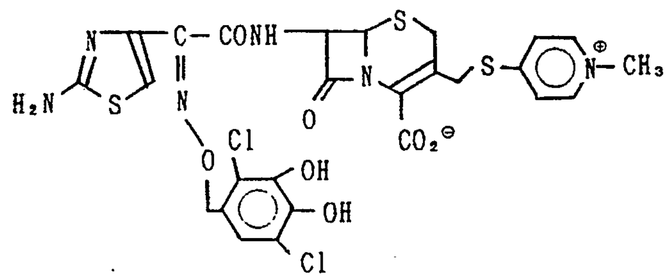

A suspension of 150mg of p-methoxybenzyl 7-[2-(2-tritylaminothiazol-4-yl)-2(Z)-(2,5-dichloro-3,4-di-p-methoxybenzyloxybenzyloxyimino)acetamido]-3-(N-methylpyridin-4-yl)thiomethyl-3-cephem-4-carboxylate•iodide (as obtained by the above B ) in 0.2ml of anisole was ice-cooled, to which 2ml of trifluoroacetic acid were added and stirred for an hour at the same temperature. After the completion of the reaction, 50ml of isopropyl ether were added to the reaction mixture to afford trifluoroacetate of the title compound as powders. The powders were washed with isopropyl ether, dried and suspended in 5ml of water. The suspension was adjusted to pH 7.2 by addition of 7% $NaHCO_3$. The resulting solution was subjected to a column of DIAION® HP-20 (20ml) eluting with 20% aqueous acetone. The eluate was concentrated under reduced pressure and freeze-dried to obtain 22mg of the title compound.
IR (nujol) 3300-3200, 1760, 1680, $1620cm^{-1}$
NMR(DMSO-$d_6$, $\delta$ value, ppm)
3.7392H,bs), 4.10(3H,s), 4.40,4.56(2H,ABq,J=14Hz), 4.95(1H,bs), 5.03(1H,d,J=4.8Hz), 5.29(1H,dd,J=4.8Hz and 9Hz), 6.63(1H,s), 6.74(1H,s), 8.28,8.58(4H, ABq,J=7Hz), 9.58(1H,d,J=9Hz)

Example 4

A. p-Methoxybenzyl 7-[2-(2-tritylaminothiazol-4-yl)-2(Z)-(2-chloro-3,4-di-p-methoxybenzyloxybenzyloxyimino)acetamido]-3-chloromethyl-3-cephem-4-carboxylate

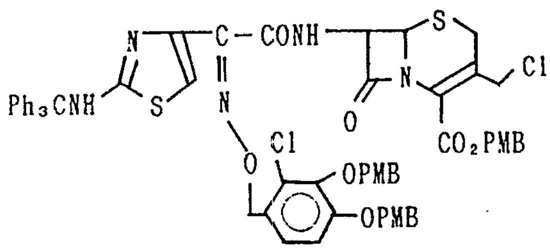

By the same method as in Example 1, the title compound (920mg) was obtained from 825mg of 2-(2-tritylaminothiazol-4-yl)-2(Z)-(2-chloro-3,4-di-p-methoxybenzyloxybenzyloxyimino)acetic acid and 370mg of p-methoxybenzyl 7-amino-3-chloromethyl-3-cephem-4-carboxylate.
IR (nujol) 3300, 1770, 1720, 1680, 1610cm$^{-1}$
NMR($CDCl_3$, $\delta$ value, ppm)
3.25,3.52(2H,ABq,J=18Hz), 3.80(9H,s), 4.40,4.50(2H,ABq,J=12Hz), 4.93(1H,d,J=5Hz), 5.2-5.25(8H,m), 5.84(1H,dd,J=5Hz and 9Hz), 6.70-6.90(9H,m), 7.25-7.35(21H,m)

Example 5

A. p-Methoxybenzyl 7-[2-(2-tritylaminothiazol-4-yl)-2(Z)-(3,4-di-p-methoxybezyloxybenzyloxyimino)-acetamido]-3-(1,2,3-thiadiazol-5-yl)thiomethy-3-cephem-4-carboxylate

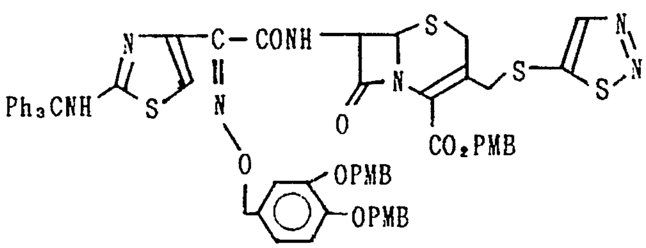

2-(2-Tritylaminothiazol-4-yl)-2(Z)-(3,4-di-p-methoxybenzyloxybenzyloxyimino)acetic acid (800mg) and p-methoxybenzyl 7-amino-3-(1,2,3-thiadiazol-5-yl)thiomethyl-3-cephem-4-carboxylate (440mg) were dissolved in 20ml of methylene chloride and cooled to -20°C. To the cooled solution were added 320mg of pyridine and then drop- wise added a solution of 240mg of phosphorus oxychloride in 5ml of methylene chloride. The mixture was stirred for an hour under ice-cooling and then poured into ice-water. The mixture was adjusted to pH 7.0, washed with water and then dried over anhydrous $MgSO_4$. After removing the solvent under reduced pressure, the residue was purified by silica gel column chromatography [Wako-gel® C-200: 30g, developing solvent : benzene and ethyl acetate (20:1)] to obtain 920mg of the title compound.
IR (nujol) 3300-3200, 1760, 1720, 1680, 1610cm$^{-1}$
NMR($CDCl_3$, $\delta$ value, ppm)
3.15,3.42(2H,ABq,J=18Hz), 3.82(6H,s), 3.86,4.16(2H,ABq,J=14Hz), 4.90(1H,d,J=5Hz), 5.10(2H,bs), 5.05-(2H,bs), 5.25(2H,bs), 5.85(1H,dd,J=5Hz and 9Hz), 6.78-7.05(9H,m), 7.30-7.45(19H,m), 8.40(1H,s)

B. Sodium 7-[2-(2-aminothiazol-4-yl)-2(Z)-(3,4- dihydroxybenzyloxyimino)acetamido]-3-(1,2,3-thiadiazol-5-yl)-thiomethyl-3-cephem-4-carboxylate

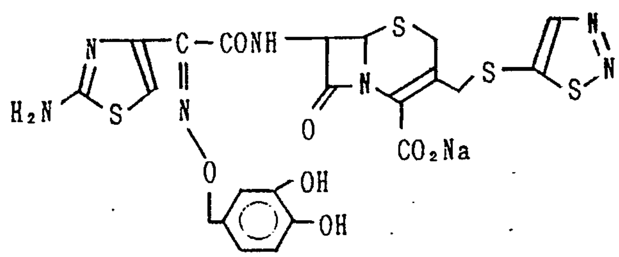

A solution of 150mg of p-methoxybenzyl 7-[2-(2-tritylaminothiazol-4-yl)-2(Z)-(3,4-di-p-methoxybenzyloxybenzyloxyimino)acetamido]-3-(1,2,3-thiadiazol-5-yl) thiomethyl-3-cephem-4-carboxylate (as obtained by the above A) in 2ml of anisole was ice-cooled, to which 2ml of trifluoroacetic acid were added and stirred for an hour at 5°C. To the reaction mixture were added 50ml of isopropyl ether, by which trifluoroacetate of the title compound were precipitated. The precipitate as collected by filtration was suspended in 1ml of water ,which was adjusted to pH 7.2 by addition of 7% $NaHCO_3$. The resulting solution was subjected to a column of DIAION® HP-20 (20ml) eluting with 10% aqueous acetone. The eluate was concentrated under reduced pressure to remove acetone and then freeze-dried to obtain 42mg of the title compound.
IR (nujol) 3300-3200, 1760cm$^{-1}$
NMR($D_2O$, $\delta$ value, ppm)
3.25,3.65(2H,ABq,J=18Hz), 4.10,4.35(2H,ABq,J=14Hz), 5.05(1H,d,J=5Hz), 5.15(2H,s), 5.72(1H,d,J=5Hz), 5.85-7.05(3H,m), 8.70(1H,s)

Example 6

Sodium 7-[2-(2-aminothiazol-4-yl)-2(Z)-(3,4-dihydroxybenzyloxyimino)acetamido]-3-(5-methyl-2-tetrazol-2-yl)-thiomethyl-3-cephem-4-carboxylate

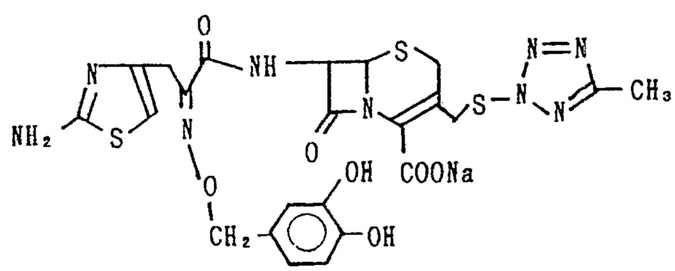

By the same method as in Example 5, the title compound (18mg) was obtained from 200mg of 2-(2-trityl aminothiazol-4-yl)-2(Z)-(3,4-dimethoxybenzyloxybenzyloxyimino)acetic acid and 120mg of diphenylmethyl 7-amino-3-(5-methyl-2H-tetrazol-2-yl)thiomethyl-3-cephem-4-carboxylate.
IR (nujol) 3300-3200, 1770cm$^{-1}$
NMR($D_2O$, $\delta$ value, ppm)
2.4(3H,s), 3.4(2H,bv.s), 5.0(1H,d,J=6Hz), 5.1(2H,s), 5.6(1H,d,J=5Hz), 5.8(2H,ABq), 5.9-7.05(3H,m)

Example 7

A: p-Methoxybenzyl 7-[2-(2-tritylaminothiazol-4-yl)-2(Z)-(5-chloro-3,4-di-p-methoxybenzyloxybenzyloxyimino)acetamido]-3-(1,2,3-thiadiazol-5-yl)thiomethyl-3-cephem-4-carboxylate

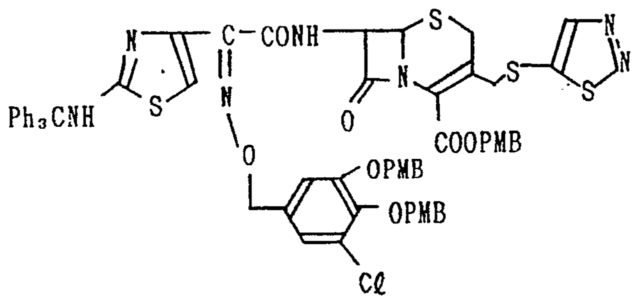

2-(2-Tritylamonithiazol-4-yl)-2(Z)-(5-chloro-3,4-di-p-methoxybenzyloxybenzyloxyimino)acetic acid (466mg) and p-methoxybenzyl 7-amino-3-(1,2,3-thiadiazol-5-yl) thiomethyl-3-cephem-4-carboxylate (260mg) were dissolved in 30ml of methylene chloride and cooled to -20° C, to which 180mg of pyridine and a solution of 100mg of phosphorus oxychloride in 2ml of methylene chloride were added. The mixture was stirred for 30 minutes at -20° C and for further 30 minutes at -10° C, and then poured into 20ml of ice-water. The mixture was adjusted to pH 7.3 by addition of 7% aqueous sodium hydrogen carbonate under stirring. The organic layer was washed with water, dried over anhydrous $MgSO_4$ and concentrated under reduced pressure. The residue was purified by silica gel column chromatography (Wako-gel® C-200, 50g; developing solvent: benzene-ethyl acetate) to obtain 631mg (91%) of the title compound.

MS m/z: 1258 $(M+H)^+$

IR (nujol): 3400, 1786, 1719, 1684, 1586cm$^{-1}$

NMR ($CDCl_3$)δ: 3.25, 3.50(2H, ABq, J=18H₂, 2-H), 3.76(6H, bs, $OCH_3$ x 3), 3.95, 4.15 (2H, ABq, J=13Hz, 3′-H), 4.85(1H, d, J=5Hz, 6-H), 4.95(2H, S, $OCH_2$), 5.05- 5.20(6H, m, $OCH_2$ x 3), 5.82(1H, dd, J=5Hz, 8Hz, 7-H), 6.75-7.40(32H, m,
arom, NH x 2, thiazole 5-H), 8.35 (1H, s, thiadiazole 4-H).

B. Sodium 7-[2-(2-aminothiazol-4-yl)-2(Z)-(5-chloro-3,4-dihydroxybenzyl)oxyimino)acetamido]-3-(1,2,3-thiadiazol-5-yl)thiomethyl-3-cephem-4-carboxylate

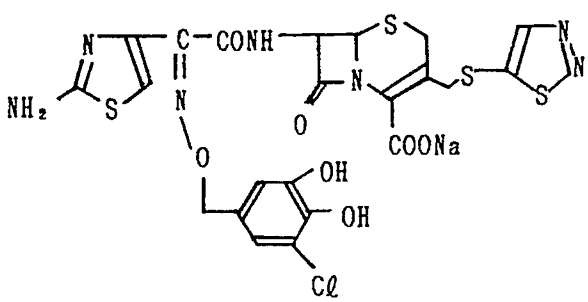

The ester (610mg) as obtained by the above A was dissolved in 2ml of anisole and ice-cooled. Trifluoroacetic acid (4ml) was added to the solution and stirred for 2 hours at 0 to 5° C. After adding 50ml of isopropyl ether, the mixture gave a precipitate which was collected by centrifugation and washed with isopropyl ethyl. The product was dissolved in a solution of 110mg of sodium hydrogen carbonate in 5ml of water, and the solution was adjusted to pH 7.5 - 7.8 by addition of 0.1N-hydrochloric acid. The mixture was treated with 60ml of Diaion® HP-20, which was washed with water and eluted with 20% aqueous acetone. The eluate was concentrated under reduced pressure to remove acetone and the residue was freeze-dried to obtain 231mg (71%) of the title compound (sodium salt).

IR (nujol): 3400-3800, 1762, 1718, 1670cm$^{-1}$

NMR(DMSO-$d_6$)δ: 3.25, 3.40(2H, ABq, J=18H₂, 2-H), 4.40(2H, bs, 3-H), 4.90(2H, s, $OCH_2$), 4.95(1H, d, J=5Hz, 6-H), 5.55(1H, dd, J-5Hz, 8Hz, 7-H), 6.65-6.75(2H, m, arom), 6.70(1H, s, thiazol 5-H), 9.05(1H, s, thiadiazole 4-H), 9.50(1H, d, J=8Hz, CONH).

Example 8

Sodium 7-[2-(2-aminothiazol-4-yl)-2(Z)-(2,5-difluoro-3,4-dihydroxybenzyloxyimino)acetamido]-3-(1,2,3-thiadiazol-5-yl)thiomethyl-3-cephem-4-carboxylate

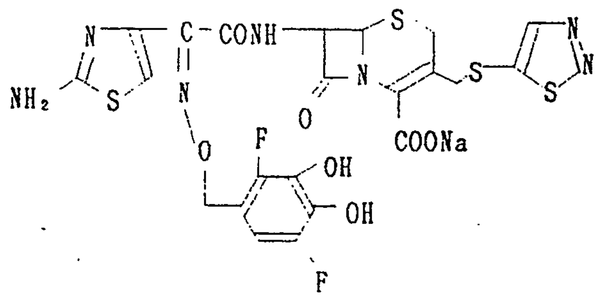

By the same method of Example 1-A, p-methoxybenzyl 7-[2-(2-tritylaminothiazol-4-yl)-2(Z)-(2,5-difluoro-3,4-di-p-methoxybenzyloxybenzyloxyimino)acetamido]-3-(1,2,3-thiadiazol-5-yl)thiomethyl-3-cephem-4-carboxylate was prepared from p-methoxybenzyl 7-amino-3-(1,2,3-thiadiazol-5-yl)thiomethyl-3-cephem-4-carboxylate and 2-(2-tritylaminothiazol-4-yl)-2(Z)-(2,5-difluoro-3,4-di-p-methoxybenzyloxybenzyloxyimino)-acetic acid. (The latter was prepared by firstly converting 2,5-difluoro-3-methoxy-4-hydroxybenzaldehyde (cf. J. Org. Chem. 46(1981), 203) into 2,5-difluoro-3,4-di-p-methoxybenzyloxybenzyl chloride via four steps and then reacting the benzyl chloride with allyl 2-(2-tritylaminothiazol-4-yl)-2(Z)-hydroxyiminoacetate.

The above p-methoxybenzyl ester compound was treated by the same way as in Example 1-B to obtain the title compound.

IR(nujol) 3400-3200, 1760, 1650, 1600$cm^{-1}$

NMR(DMSO-$d_6$, δ value, ppm)

3.60(2H,s), 4.40(2H,s), 4.98(1H,d,J=5Hz), 5.05(2H,b,s), 5.56(1H,d,d,J=5Hz and 10Hz), 6.74(1H,s), 6.88-(1H,s), 7.20(2H,b.s), 9.04(1H,s), 9.60(1H,d,J=10Hz)

Example 9

Sodium 7-[2-(2-aminothiazol-4-yl)-2(Z)-(5-fluoro-3,4-dihydroxybenzyloxyimino)acetamido]-3-(l,2,3-thiadiazol-5-yl)thiomethyl-3-cephem-4-carboxylate

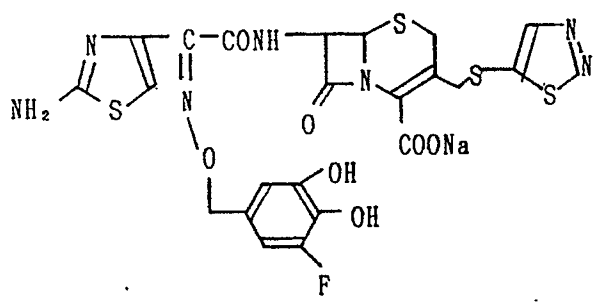

By the same method of Example 1-A, p-methoxybenzyl 7-[2-(2-tritylaminothiazol-4-yl)-2(Z)-(5-fluoro-3,4-p-methoxybenzyloxybenzyloxyimino)acetamido]-(1,2,3-thiazol-5-yl)thiomethyl-3-cephem-4-carboxylate was prepared from p-methoxybenzyl 7-amino-3-(1,2,3-thiadiazol-5-yl) thiomethyl-3-cephem-4-carboxylate and 2-(2-tritylaminothiazol-4-yl)-2(Z)-(5-fluoro-3,4-di-p-methoxybenzyloxybenzyloxyimino)acetic acid. {The latter 5-fluoro compound was prepared from 3-methoxy-4-hydroxy-5-fluorobenzaldehyde [J. Org. Chem. 51 (1986), 4073]}

The above methoxybenzyl ester compound was treated in the same way as in Example 1-B to obtain the title compound.

IR (nujol): 3400-3800, 1762, 1718, 1670$cm^{-1}$

NMR(DMSO-$d_6$)δ: 3.25, 3.40(2H, ABq, J=18$H_z$, 2-H), 4.40(2H, bs, 3-H), 4.90(2H, s, $OCH_2$), 4.95(1H, d,

J = 5Hz, 6-H), 5.55(1H, dd, J-5Hz, 8Hz, 7-H), 6.65-6.75(2H, m, arom), 6.70(1H, s, thiazol 5-H), 9.05(1H, s, thiadiazole 4-H), 9.50(1H, d, J = 8Hz, CONH).

The following Table 1 show minimum inhibitory concentrations (MIC) against Gram-positive and Gram-negative bacteria of representative compounds of the invention. MIC was measured by agar dilution method.

Table 1

Minimum Inhibitor Concentration (MIC)

| Compound / Micro-organism | Example 5 | Example 6 | Example 1 | Example 2 | Example 3 | Example 8 | Example 9 | Example 7 |
|---|---|---|---|---|---|---|---|---|
| 1) Staph. aureus Smith | 0.10 | 0.10 | 0.10 | 0.20 | 0.39 | 0.10 | 0.10 | 0.01 |
| 2) Staph. epidermidis | 0.78 | 1.56 | 0.20 | 0.20 | 0.39 | 0.20 | 0.78 | 0.78 |
| 3) Bacillus subtilis | 0.39 | 0.39 | 0.78 | 0.20 | 0.78 | 1.56 | 1.56 | 1.56 |
| 4) Escherichia coli NIHJ | 0.05 | 0.05 | 0.05 | 0.78 | 0.05 | < 0.05 | < 0.05 | < 0.05 |
| 5) Proteus mirabilis | 0.10 | 0.10 | 0.05 | 0.05 | 0.025 | < 0.05 | < 0.05 | < 0.05 |
| 6) Klebsiella pneumoniae | 0.05 | 0.10 | 0.05 | 0.20 | 0.05 | < 0.05 | < 0.05 | < 0.05 |
| 7) Salmonella typhi | 0.10 | 0.20 | 0.10 | 0.20 | 0.39 | < 0.05 | < 0.05 | < 0.05 |
| 8) Enterobacter cloacae | 0.78 | 1.56 | 0.20 | 0.78 | 0.20 | 3.13 | 6.25 | 6.25 |

Table 1 (continued)

Minimum Inhibitor Concentration (MIC)

| Compound / Micro-organism | Example 5 | Example 6 | Example 1 | Example 2 | Example 3 | Example 8 | Example 9 | Example 7 |
|---|---|---|---|---|---|---|---|---|
| 9) Serratia marcescens | 0.10 | 0.20 | 0.10 | 0.78 | 0.05 | < 0.05 | < 0.05 | < 0.05 |
| 10) Pseudomonas aeruginosa GN | 0.78 | 1.56 | 0.39 | 6.25 | 0.39 | 0.78 | 1.56 | 1.56 |
| 11) Pseudomonas aeruginosa | 0.39 | 0.78 | 0.39 | 0.78 | 0.39 | 0.39 | 0.78 | 0.78 |

## Claims

1. A cephalosporin compound of the formula (I):-

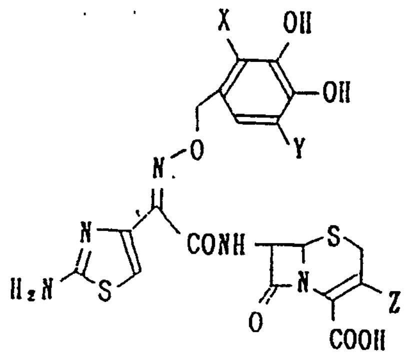

( I )

wherein X and Y may be the same or different and are hydrogen or halogen atom; Z is hydrogen, halogen, $-CH=CH_2$, $-CH=CH-CH_3$, $-OR^1$, $-SR^2$ or $-CH_2W$ in which $R^1$ and $R^2$ are a lower alkyl group and W is hydrogen or a nucleophilic residue.

2. A compound as claimed in Claim 1 in which X and Y are identical and are chlorine or fluorine.

3. A compound as claimed in Claim 1 in which one of X and Y is chlorine or fluorine and the other is hydrogen.

4. A compound as claimed in Claim 1 in which X and Y are both hydrogen.

5. A compound of claim 1 in which Z is (1,2,3-thiadiazol-5-yl)thiomethyl, (5-methyl-2H-tetrazol-2-yl)-thiomethyl or (N-methylpyridin-4-yl)thiomethyl.

6. A compound of claim 1 which is 7-[2-(2-aminothiazol-4-yl)-2(Z)-(2,5-dichloro-3,4-dihydroxybenzyloxyimino)acetamido]-3-(1,2,3-thiadiazol-5-yl)thiomethyl-3-cephem-4-carboxylic acid, 7-[2-(2-aminothiazol-4-yl)-2(Z)-(2-chloro-3,4-dihydroxybenzyloxyimino)acetamido]-3-(1,2,3-thiadiazol-5-yl)thiomethyl-3-cephem-4-carboxylic acid, 7-[2-(2-aminothiazol-4-yl)-2(Z)-(2,5-dichloro-3,4-dihydroxybenzyloxyimino)acetamido]-3-(N-methylpyridin-4-yl)thiomethyl-3-cephem-4-carboxylic acid, 7-[2-(2-(aminothiazol-4-yl)-2(Z)-(3,4-dihydroxybenzyloxyimino)acetamido]-3-(1,2,3-thiazol-5-yl)thiomethyl-3-cephem-4-carboxylic acid, 7-[2-(2-aminothiazol-4-yl)-2(Z)-(3,4-dihydroxybenzyloxyimino)acetamido]-3-(5-methyl-2H-tetrazol-2-yl)thiomethyl-3-cephem-4-carboxylic acid, 7-[2-(2-aminothiazol-4-yl)-2(Z)-(5-chloro-3,4-dihydroxyphenzyloxyimino)-acetamido]-3-(1,2,3-thiadiazol-5-yl)thiomethyl-3-cephem-4-carboxylic acid, 7-[2-(2-aminothiazol-4-yl)-2(Z)-(2,5-difluoro-3,4-dihydroxybenzyloxyimino)acetamido]-3-(1,2,3-thiadiazol-5-yl)thiomethyl-3-cephem-4-carboxylic acid or 7-[2-(2-aminothiazol-4-yl)-2(Z)-(5-fluoro-3,4-dihydroxybenzyloxyimino)acetamido[-3-(1,2,3-thidiazol-5-yl) thiomethyl-3-cephen-4-carboxylic acid, or a salt thereof.

7. A pharmaceutically acceptable salt of a cephalosporin compound as claimed in any preceding claim.

8. Use of a therapeutically-active substance for the preparation of a medicament for the therapy of disorders caused in human subjects by Pseudomonas bacterial strains or beta-lactamase-producing bacterial strains, characterized in that the therapeutically-active substance is a cephalosporin compound as claimed in any one of Claims 1 to 6 or a salt thereof as claimed in Claim 7.

9. An antibacterial composition comprising an effective amount of a cephalosporin compound as claimed in any one of Claims 1 to 6, or a salt thereof as claimed in Claim 7, together with a pharmaceutically acceptable carrier or diluent.

European Patent Office

# EUROPEAN SEARCH REPORT

Application number

EP 90304470.9

| Category | DOCUMENTS CONSIDERED TO BE RELEVANT<br>Citation of document with indication, where appropriate, of relevant passages | Relevant to claim | CLASSIFICATION OF THE APPLICATION (Int Cl 5) |
|---|---|---|---|
| X | EP - A2/A3 - 0 241 901 (BANYU PHARMACEUTICA CO. LTD.)<br>* examples 14,33,38; claims; especially claim 2 (12)(31)(45) * | 1,4-9 | C 07 D 501/20<br>C 07 D 501/22<br>C 07 D 501/36<br>A 61 K 31/545 |
| Y | EP - A2 - 0 280 521 (GLAXO)<br>* Examples; claims * | 1,7-9 | |
| Y | EP - A2 - 0 251 299 (KAKEN)<br>* Claims; table 2 * | 1,7-9 | |
| A | EP - A2 - 0 007 633 (FUJISAWA)<br>* Abstract; example 14 * | 1,7-9 | |
| A | GB - A - 2 197 649 (GLAXO)<br>* claims; page 9, last line * | 1-9 | TECHNICAL FIELDS SEARCHED (Int Cl 5) |
| A | EP - A2/A3 - 0 178 527 (KAKEN)<br>* Abstract; table 1 * | 1-9 | C 07 D 501/00 |

The present search report has been drawn up for all claims

| Place of search | Date of completion of the search | Examiner |
|---|---|---|
| VIENNA | 01-08-1990 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82